# EUROPEAN PATENT SPECIFICATION

(11) **EP 2 723 236 B1**
(45) Date of publication and mention of the grant of the patent: **18.03.2015**
(21) Application number: 12740698.1
(22) Date of filing: 26.06.2012
(51) Int. Cl.: A61B 5/15, A61B 5/153, A61B 5/154

(54) **Holder with collection chamber for blood samples**
Halter mit Sammelkammer für Blutproben
Support ayant chambre de collecte pour des échantillons de sang

(30) Priority: 27.06.2011 IT RM20110338
(43) Date of publication of application: 30.04.2014
(73) Proprietor: Pepe, Mauro, 00167 Roma (IT)
(72) Inventor: Pepe, Mauro, 00167 Roma (IT)
(74) Representative: Fiammenghi, Eva
(86) International application number: PCT/IB2012/053225
(87) International publication number: WO 2013/001453

(56) References cited:
- EP-A1- 1 027 864
- WO-A1-89/10723
- WO-A1-2004/062499
- WO-A1-2005/041773
- WO-A1-2005/087102
- AU-A1- 2003 268 869
- US-A- 4 416 290
- US-A1- 2006 015 037
- US-A1- 2008 167 577

## Description

### Field of the art

The present patent application generally refers to the health field and more particularly to the hematological field.

The invention is applicable to any field where one such device type can be advantageously used, but preferably this regards the field of blood sampling.

### State of the art

Hematochemical exams require, in nearly all cases, the execution of a venous blood sampling; this represents a required passage for obtaining the biological matrix to be analyzed. Blood sampling is one of the oldest medical practices, used since antiquity.

The collection of venous blood, obtained through the incision of a vein with a lancet, has been described since the days of Hippocrates (5^{th} century BC). During the Middle Ages, barber-surgeons were specialized in this practice; indeed, the white-red pole of the barber professions, still in use today in some countries, derives from this activity. In the 18th and 19th centuries, venous blood collection reached a considerable diffusion, using a variety of methods. The most common technique is based on the so-called phlebotomy or venisection, wherein the blood was collected after the incision of one or more large external veins of the forearm or of the neck. However, it was only at the beginning of the 20th century, after the introduction of needles and syringes, that venous blood sampling made use of less invasive and more reliable techniques. A further innovation came immediately after the end of the Second World War, when Becton Dickinson during the early 1950s introduced the first "Vacutainer" vacuum systems.

Currently, two main analysis systems can be provided for blood sampling: that with disposable needle and syringe and subsequent filling of test tubes via transfer, and that which employs vacuum systems that allow the direct collection of the blood in the test tube via controlled suction. Ignoring the syringe and needle analysis system - by now obsolete and reserved to very particular cases, or emergency cases where it is not possible to procure other suitable devices -, today the use of vacuum sampling systems is particularly recommended; such vacuum systems have numerous characteristics that ensure the quality of the sample. The aforesaid vacuum sampling system is constituted by test tubes of various capacity made of glass or plastic, in which a pre-calibrated vacuum has been created corresponding to the quantity of blood to be drawn; by a two-tipped needle, one tip for puncturing the vein and the other for perforating the cap of the test tubes themselves; and by a plastic holder or jacket, in which the needle is screwed on one side and the test tube to be filled is inserted on the other side. The closed system characteristic typical of the vacuum, which eliminates the risk of contact of the sampling technician with the blood of the patient, translates into considerable safety for the reduction of the biological risk.

However, notwithstanding the many benefits of the vacuum sampling system, even such system has several disadvantages. In particular, as all those skilled in the art know, there is a particular difficulty, mainly due to the intrinsic characteristics of the aforesaid device constituted by the needle, the holder and the test tube, in specifically and directly controlling the correct flow of the blood inside the device itself. In reality, such aspect is rather important, since the ready analysis of such situation allows the sampling technician (even one less expert) to immediately verify the correct position of the needle in the vein as well as a possible collapse of the vessel, and hence verify the correct execution of the sampling itself.

Hence, the object of the present invention is to make a so-called "holder with collection chamber for blood samples" with specific characteristics which render it directly usable with the most common sampling devices currently present on the market, inventively improving several specific characteristics of such devices, as will be clear from the description of an exemplifying and non-limiting embodiment thereof illustrated below.

### Description of the invention

The innovative concept underlying the present invention mainly consists of providing for a modified holder for blood samples that is capable of facilitating the operations connected with the blood sampling, nevertheless maintaining unchanged all the positive characteristics regarding the standardization of the operative procedure and the lower risk of biological contamination for the operator, already part of the prior art.

More particularly, the present invention tends to achieve a holder for blood samples equipped with an inventive collection chamber interposed between the venipuncture needle and the test tube perforation needle. In this manner, a device is obtained capable of ensuring a ready observation of the correct execution of the blood drawing and hence an entire series of advantages with respect to current systems. More specifically, said inventive collection chamber, interposed between the venipuncture needle and the test tube perforation needle, specifically and directly allows observing the correct flow of the blood inside the device itself. In reality, such aspect is rather important, since the ready verification of such situation allows the sampling technician (even one less expert) to immediately verify the correct position of the needle in the vein as well as a possible collapse of the vessel, and hence verify the correct and rapid execution of the sampling itself. Still more specifically, the stasis and increase of the venous pressure that is often observed in a non-optimal and/or overly prolonged sampling involve the possibility of exchange between the plasma and erythrocytes, the increase of the acidity of the blood due to some acid metabolites produced by the tissue metabolism, the consequent displacement of some substances from their bond with the transport proteins and hence a particularly altered hematochemical scenario. Another technical characteristic which differentiates the holder, object of the present invention, is that due to the particular morphology it is perfectly adapted to the requirements connected to an efficient sampling, even by less expert operators. In particular, the particular external conformation, with one face flattened, of the embodiment of the invention, or with the axis of the needle arranged in eccentric position with respect to the axis of said inventive holder, allows easily obtaining the correct tilt of the needle with respect to the arm of the patient, i.e. obtain a low tilt degree of the needle, equal to about 10° with respect to the vein. With regard to the materials constituting said inventive holder, these can be selected by the man skilled in the art in order to meet the technical needs in this particular field, and which therefore must meet all the strict safety and procedural norms regarding sanitary devices. In particular, said inventive holder is a strictly disposable product, to be used in accordance with the use procedures in place in sanitary/health structures; it is sterile, separately packaged and constituted by polypropylene or by any other suitable thermoplastic polymer. In addition, said inventive holder can be made of totally transparent and/or translucent and/or opaque material, nevertheless considering that, for the inventive specificity, the aforesaid collection chamber must be exclusively made of transparent and/or translucent material. With regard to the size of said holder, it should be underlined that this does not limit the invention, since the constituent technical characteristics render it adaptable to the specific use and mainly to the size of the secondary devices already in use on the market (test tubes, vacuum test tubes, phleboclysis containers, puncture needles, etc.). Nevertheless, in an embodiment intended for neonatal and adults patients, the size regarding said inventive collection chamber can be quantitative summarized as a volume comprised between 6 ml and 0.5 ml, more preferably comprised between 4.5 ml and 1 ml, and still more preferably is 3 ml. In addition, with regard to an embodiment intended for different uses, the size regarding said inventive collection chamber can be quantitatively summarized as a volume comprised between 15 ml and 3 ml, more preferably comprised between 12 ml and 6 ml, and still more preferably is 10 ml.

Other characteristics of the present invention are described in the following detailed description of one or more specific embodiments, protected by the various dependent claims.

### Brief description of the drawings

The preceding advantages, as well as other advantages and characteristics of the present invention, will be illustrated by making reference to the enclosed figures, which are to be considered merely illustrative and non-limiting or non-binding with regard to the present patent application, in which:
FIGURE 1 is a lateral view of the holder according to the present invention;
FIGURE 2 is a lateral perspective view of the holder according to the present invention;
FIGURE 3 is a top plan view of the holder according to the present invention;
FIGURE 4 is a lateral perspective view of the holder according to an alternative embodiment of the present invention.

As previously stated, it should be underlined that the borders, shapes and areas shown in the aforesaid figures must not be considered as limiting the invention, as this is preferably directed to any type, size, diameter, color, translucence and shape so long as it does not depart from the inventive concept.

### Preferred embodiments of the invention

The present invention will now be described in detail by making reference to the figures and to a preferred embodiment shown therein, in which identical numeric references were used for the same components.

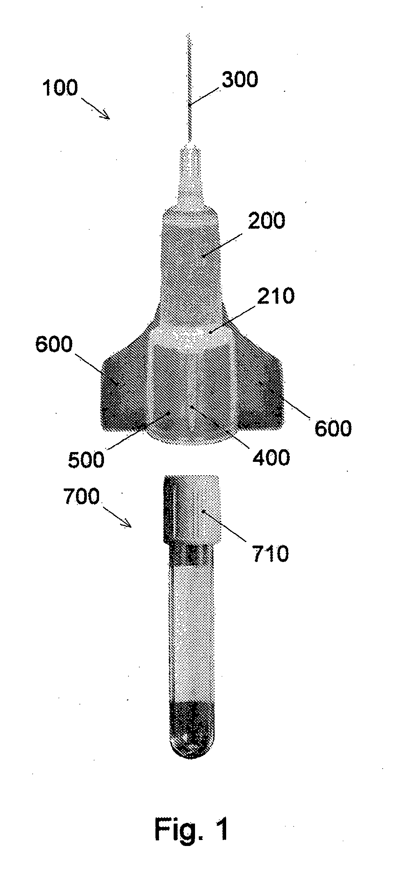

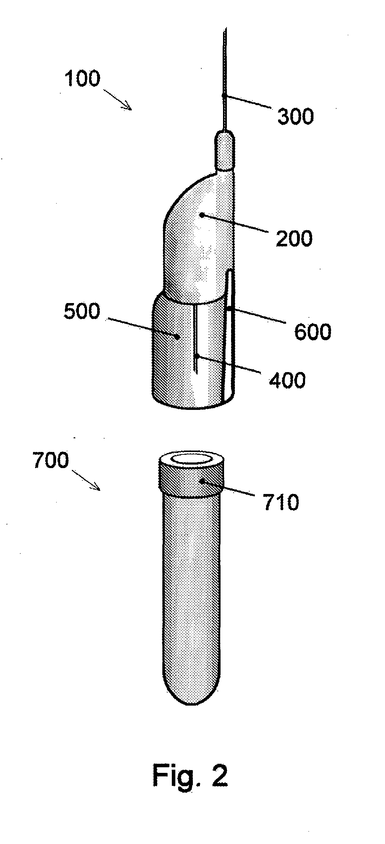

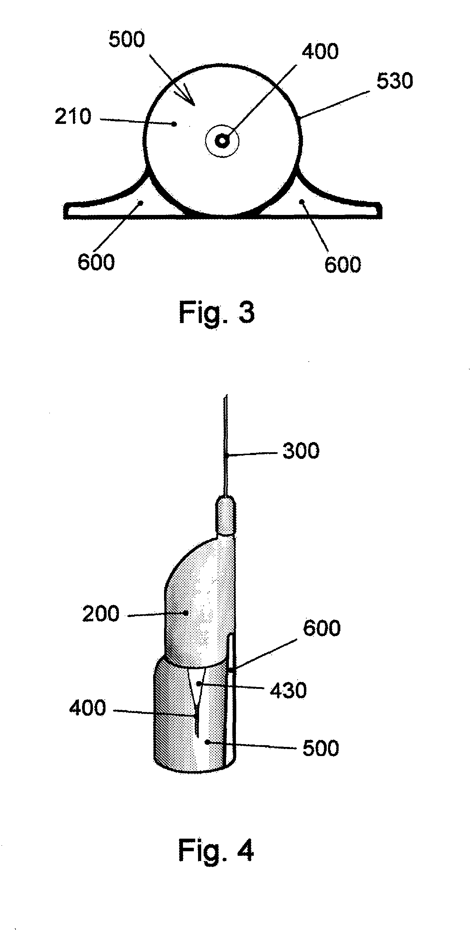

As shown in Figures 1 and 2, said inventive holder 100 centrally comprises a collection chamber 200 that is roughly cylindrical and tapered at the upper part at the insertion of a common steel puncture needle 300 equipped with a classic thread and placed in eccentric position with respect to the axis of said collection chamber 200. Such inventive collection chamber 200 also results in communication on a lower part with a test tube housing seat 500, exclusively by means of a test tube perforation needle 400 made of plastic material, centrally placed (see Fig. 3) on the base 210 of said collection chamber 200. As can be specifically observed in Fig. 3, such test tube housing seat 500 is constituted by the aforesaid base 210 and by a lateral wall 530 created on the periphery of the aforesaid base 210 and wherein the length of said lateral wall 510 is greater than that of said test tube perforation needle 400. More specifically, as results from Figures 1, 2 and 3, said collection chamber 200 has, close to the base, the test tube housing seat 500 in direct communication with said collection chamber 200 only by means of the test tube perforation needle 400; said test tube housing seat 500 is such to allow the direct insertion and then the perforation of the cap 710 of a common test tube or of a vacuum test tube 700 executed by said test tube perforation needle 400.

On the rear-lateral side of said collection chamber 200 and of the test tube housing seat 500, at least two finger-rest tabs 600 are present, which as can be observed in Figs. 2 and 3 are co-planar, as much as possible, with said puncture needle 300, in a manner so as to achieve overall a flat rear face of the inventive holder 100.

The use mode of said inventive holder 100 results rather intuitive, since once the hemostatic lace is applied to the arm (only for the time necessary to identify the vein to be punctured), one easily carries out (also by means of gripping the two finger-rest tabs 600) the venipuncture with the puncture needle 300 screwed on the collection chamber 200. At this point, due to the venous pressure, the blood should start to flow into the proper collection chamber 200 up to the requested quantity. Once the filling has been completed, the inventive holder 100 will be extracted, with the blood collected in the relative collection chamber 200 and at this point a common test tube or better yet vacuum test tube 700 will be conveniently inserted in the proper test tube housing seat 500, in a manner such that the test tube perforation needle 400 perforates the cap 710 of the test tube itself. After this maneuver, a direct communication is created between the collection chamber 200 and the vacuum test tube 700 interior and hence a constant blood quantity, due to the action of the reduced pressure present in the test tube itself, will be sucked inside the latter until the vacuum is depleted. Once the filling is completed, one can extract said test tube in order to fill other test tubes. A further use mode provides that the aforesaid insertion of the series of vacuum test tubes 700 in the suitable test tube housing seat 500 occurs when the puncture needle 300 is still in the vein, facilitating, by means of the reduced pressure created by the same vacuum test tube 700, the passage of the blood itself from the vein into the said collection chamber 200 and from this into the aforesaid vacuum test tube 700. Nevertheless, in both use modes, the filling of the vacuum test tube 700 occurs in an easy and constant manner, the chemical cleaning and the sterility of the internal space are maintained unaltered, it is possible to fill multiple test tubes by means of a single venipuncture and finally it is easy to insert the needle in the vein since low tilt angles can be reached, due to the particular morphology of the inventive holder - in the inventor's opinion, all of the above indicate that the present invention is the best means for drawing venous blood as well as other organic fluids that is currently present on the market.

A further embodiment of the present invention provides that in the collection chamber 200, the common chemical additives necessary for stabilizing the sample and for carrying out the hematochemical exams can be previously inserted. In addition, it is provided that said collection chamber 200 is made of reversibly compressible or elastic materials in order to create a zone of reduced pressure inside the collection chamber 200. Said reduced pressure is created by compressing the same collection chamber 200 with one's finger.

The present invention provides for further alternative embodiments corresponding with several variants of its structure and of the shape of its components. For example, even if up to now the puncture needle 300 has been described that is adapted to perforate only the caps 710 of the most common test tubes or vacuum test tubes 700, it is possible to extend the field of application of the present invention by creating (as can be inferred in Fig. 4) around the base of said test tube perforation needle 400 a further conical engagement 430 of plastic material useful for perforating the cap even of the common phleboclysis containers.

The present invention has been described as an illustrative, non-limiting example, according to several preferred embodiments, but it is intended that possible variations and/or modifications can be made by men skilled in the art without departing from the relative protective scope, as defined by the following dependent claims.

## Claims

1. Holder with collection chamber for blood samples, **characterized in that** said holder (100) centrally comprises a collection chamber (200) that is roughly cylindrical and tapered at the upper part at the insertion of a common steel puncture needle (300) equipped with a common thread and placed in eccentric position with respect to the axis of said collection chamber (200), having said collection chamber (200) in communication on its lower part with a test tube housing seat (500), by means of a test tube perforation needle (400) made of plastic material, centrally placed on the base (210) of said collection chamber (200), and said test tube housing seat (500) being constituted by the aforesaid base (210) and by a lateral wall (530) created on the periphery of the aforesaid base (210) and in which the length of said lateral wall (530) is greater than that of said test tube perforation needle (400), and having said test tube housing seat (500) made in a manner such to allow the direct insertion and then the perforation of a cap (710) of a common test tube or of a vacuum test tube (700) executed by said test tube perforation needle (400) and having the rear-lateral face of said collection chamber (200) flat and the underlying test tube housing seat (500) equipped with at least two finger-rest tabs (600) that are co-planar, as much as possible, with said puncture needle (300), in a manner so as to achieve an overall flat rear surface of the holder (100).

2. Holder according to claim 1, **characterized in that** around the base of said test tube perforation needle (400), a conical engagement (430) of plastic material is created that can be used to perforate the cap even of the common phleboclysis containers.

3. Holder according to any one of the preceding claims, **characterized in that** it can be used for sampling organic fluids.

4. Holder according to any one of the preceding claims, **characterized in that** it can be a disposable, sterile and separately-packaged product.

5. Holder according to any one of the preceding claims, **characterized in that** it has the collection chamber (200) made of reversibly compressible or elastic material in a manner so as to create reduced pressure at its interior.

6. Holder according to any one of the preceding claims, **characterized in that** it can be constituted by a thermoplastic polymer.

7. Holder according to any one of the preceding claims, **characterized in that** the collection chamber (200) is made of transparent and/or translucent material.

8. Holder according to any one of the preceding claims, **characterized in that** the size of said collection chamber (200) is quantitatively summarized as a volume comprised between 6 ml and 0.5 ml, more preferably comprised between 4.5 ml and 1 ml, and still more preferably is 3 ml.

9. Holder according to any one of the preceding claims, **characterized in that** the size of said collection chamber is quantitatively summarized as a volume comprised between 15 ml and 3 ml, more preferably comprised between 12 ml and 6 ml, and still more preferably is 10 ml.

10. Holder according to any one of the preceding claims, **characterized in that** the common chemical additives necessary for stabilizing the sample and for carrying out the hematochemical exams are previously inserted in the collection chamber (200).

## Patentansprüche

1. Halter mit Sammelkammer für Blutproben, **dadurch gekennzeichnet, dass** der Halter (100) mittig eine Sammelkammer (200) aufweist, die ungefähr zylindrisch ist und im oberen Teil an der Einführung einer üblichen Stahleinstichnadel (300) konisch ist, die mit einem üblichen Gewinde versehen ist und in Bezug auf die Achse der Sammelkammer (200) in einer exzentrischen Position angeordnet ist, wobei die Sammelkammer (200) in ihrem unteren Teil mittels einer Teströhrchendurchstechnadel (400) mit einem Teströhrchengehäusesitz (500) in Verbindung steht, die aus Kunststoffmaterial besteht, mittig auf der Basis (210) der Sammelkammer (200) angeordnet ist, und wobei der Teströhrchengehäusesitz (500) aus der genannten Basis (210) und einer Seitenwand (530) besteht, die am Außenrand der genannten Basis (210) erzeugt ist, und wobei die Länge der Seitenwand (530) größer ist als die der Teströhrchendurchstechnadel (400), und wobei der Teströhrchengehäusesitz (500) auf solche Weise gefertigt ist, dass er die direkte Einführung und anschließende Perforation des Deckels (710) eines üblichen Teströhrchens oder eines Vakuumteströhrchens (700), die von der Teströhrchendurchstechnadel (400) durchgeführt wird, gestattet, und die hintere seitliche Fläche der Sammelkammer (200) flach ist und der darunter liegende Teströhrchengehäusesitz (500) mit mindestens zwei Fingerauflageplättchen (600) versehen ist, die so weit wie möglich coplan sind mit der Einstechnadel (300), so dass eine insgesamt flache hintere Fläche des Halters (100) erhalten wird.

2. Halter nach Anspruch 1, **dadurch gekennzeichnet, dass** um die Basis der Teströhrchendurchstechnadel (400) eine konische Angriffseinrichtung (430) aus Kunststoffmaterial erzeugt ist, die verwendet werden kann, um sogar die Kappe der üblichen Phleboklyse-Behälter zu durchstechen.

3. Halter nach einem der vorangehenden Ansprüche, **dadurch gekennzeichnet, dass** er zur Entnahme von Proben organischer Fluide verwendet werden kann.

4. Halter nach einem der vorangehenden Ansprüche, **dadurch gekennzeichnet, dass** er ein steriles und separat verpacktes Einwegprodukt sein kann.

5. Halter nach einem der vorangehenden Ansprüche, **dadurch gekennzeichnet, dass** die Sammelkammer (200) aus reversibel komprimierbarem oder elastischem Material besteht, so dass in ihrem Inneren ein verminderter Druck erzeugt wird.

6. Halter nach einem der vorangehenden Ansprüche, **dadurch gekennzeichnet, dass** er aus einem thermoplastischen Polymer bestehen kann.

7. Halter nach einem der vorangehenden Ansprüche, **dadurch gekennzeichnet, dass** die Sammelkammer (200) aus transparentem und/oder durchscheinendem Material besteht.

8. Halter nach einem der vorangehenden Ansprüche, **dadurch gekennzeichnet, dass** die Größe der Sammelkammer (200) quantitativ als Volumen zusammengefasst wird, das zwischen 6 ml und 0,5 ml, stärker bevorzugt zwischen 4,5 ml und 1 ml und noch stärker bevorzugt bei 3 ml liegt.

9. Halter nach einem der vorangehenden Ansprüche, **dadurch gekennzeichnet, dass** die Größe der Sammelkammer quantitativ als Volumen zusammengefasst wird, das zwischen 15 ml und 3 ml, stärker bevorzugt zwischen 12 ml und 6 ml und noch stärker bevorzugt bei 10 ml liegt.

10. Halter nach einem der vorangehenden Ansprüche, **dadurch gekennzeichnet, dass** die üblichen chemischen Zusätze, die zur Stabilisierung der Probe und zur Durchführung der hämatochemischen Untersuchungen nötig sind, zuvor in die Sammelkammer (200) eingeführt werden.

## Revendications

1. Support avec une chambre de collecte pour des échantillons de sang, **caractérisé en ce que** le support (100) comprend de manière centrale une chambre de collecte (200) qui est approximativement cylindrique et qui est effilée sur sa partie supérieure, au niveau de l'entrée d'une aiguille de ponction en acier courante (300) pourvue d'un filetage courant et placée dans une position excentrique par rapport à l'axe de la chambre de collecte (200), ladite chambre de collecte (200) communiquant, sur sa partie inférieure, avec un appui de logement d'éprouvette (500), à l'aide d'une aiguille de perforation d'éprouvette (400) en matière plastique, placée de manière centrale sur la base (210) de la chambre de collecte (200), l'appui de logement d'éprouvette (500) étant constitué par ladite base (210) et par une paroi latérale (530) créée sur la périphérie de la base (210), la longueur de la paroi latérale (530) étant supérieure à celle de l'aiguille de perforation d'éprouvette (400), l'appui de logement d'éprouvette (500) étant réalisé de manière à permettre l'introduction directe, puis la perforation d'un couvercle (710) d'une éprouvette courante ou d'une éprouvette à vide (700) exécutée par ladite aiguille de perforation d'éprouvette (400), et la face latérale arrière de la chambre de collecte (200) étant plate et l'appui de logement d'éprouvette (500) situé au-dessous étant équipé d'au moins deux pattes d'appui pour les doigts (600) qui sont coplanaires, autant que possible, par rapport à l'aiguille de ponction (300), de manière à former une surface arrière totale plate pour le support (100).

2. Support selon la revendication 1, **caractérisé en ce que**, autour de la base de l'aiguille de perforation d'éprouvette (400), un accouplement conique (430) de matière plastique est créé, qui peut être utilisé pour perforer le couvercle même des récipients pour phléboclyse courants.

3. Support selon l'une quelconque des revendications précédentes, **caractérisé en ce qu'**il peut être utilisé pour le prélèvement de fluides organiques.

4. Support selon l'une quelconque des revendications précédentes, **caractérisé en ce qu'**il peut s'agir d'un produit jetable, stérile et emballé séparément.

5. Support selon l'une quelconque des revendications précédentes, **caractérisé en ce que** sa chambre de collecte (200) est en matériau compressible de manière réversible ou élastique, de manière à créer une pression réduite, à l'intérieur.

6. Support selon l'une quelconque des revendications précédentes, **caractérisé en ce qu'**il peut être constitué par un polymère thermoplastique.

7. Support selon l'une quelconque des revendications précédentes, **caractérisé en ce que** la chambre de collecte (200) est en matériau transparent et/ou translucide.

8. Support selon l'une quelconque des revendications précédentes, **caractérisé en ce que** la taille de la chambre de collecte (200) se résume quantitativement à un volume compris entre 6 ml et 0,5 ml, de préférence entre 4,5 ml et 1 ml, et est plus spécialement de 3 ml.

9. Support selon l'une quelconque des revendications précédentes, **caractérisé en ce que** la taille de la chambre de collecte se résume quantitativement à un volume compris entre 15 ml et 3 ml, de préférence entre 12 ml et 6 ml, et est plus spécialement de 10 ml.

10. Support selon l'une quelconque des revendications précédentes, **caractérisé en ce que** les additifs chimiques courants nécessaires pour stabiliser l'échantillon et pour réaliser les examens hématochimiques sont introduits au préalable dans la chambre de collecte (200).
